# EUROPEAN PATENT SPECIFICATION

(11) **EP 1 520 564 B1**
(45) Date of publication and mention of the grant of the patent: **14.04.2010**
(21) Application number: 04256048.2
(22) Date of filing: 30.09.2004
(51) Int. Cl.: A61F 5/00

(54) **Medical segmented gastric band**
Segmentiertes medizinisches Magenband
Ceinture gastrique medicale segmentée

(30) Priority: 30.09.2003 US 676946
(43) Date of publication of application: 06.04.2005
(73) Proprietor: ETHICON ENDO-SURGERY, Cincinnati,Ohio 45242 (US)
(72) Inventor: Jambor, Kristin, Cincinnati Ohio 45208 (US); Byrum, Randy, Milford Ohio 45150 (US)
(74) Representative: Tunstall, Christopher Stephen

(56) References cited:
- WO-A1-96/25130
- US-A- 4 399 809
- US-A- 4 428 365
- US-A- 4 708 140
- US-A- 4 982 731
- US-A- 5 152 770
- US-A- 5 454 831
- US-A1- 2001 018 577
- US-A1- 2004 162 460

## Description

### Background of the Invention

### Field of the Invention:

The present invention generally relates to a device for the treatment of morbid obesity. More particularly, the present invention relates to an easily insertable and removable gastric banding device that encircles and compresses a portion of the stomach thereby forming a stoma opening having a reduced diameter.

Over the years many methods of treating morbid obesity have been undertaken. One of the more promising methods employs the placement of a circumscribing band around a portion of the stomach whereby the stomach may be compressed thereby creating a stoma opening that is smaller than the normal interior diameter of the stomach thereby restricting food intake into the lower digestive portion of the stomach.

Such a band has been described in US 4,592,339 which teaches a stoma-adjustable gastric band that includes a balloon section that is expandable and deflatable through a remote injection site. The balloon expandable section adjusts the size of the stoma opening both intraoperatively and post-operatively. US 5,152,770 and US 4,399,809 both disclose implantable devices that include a flexible, elongate plate, a plurality of communicating bulbs which are arranged on one side of the plate and have a variable volume, and a connection for supplying fluid to the bulbs. US 4,428,365 discloses an anti-incontinent prosthesis comprising a support member formable into at least one generally spiral turn and a plurality of inflatable chambers disposed on the turn and connected to a pump means for applying fluid pressure to the chambers.

During the last several years, manufacturers of prior art bands have improved the designs of the balloons of these bands. One significant area of further improvement, however, is the development of a stoma-adjustable gastric band that includes a balloon section that conforms to the patient's anatomy even better than prior art bands. Such an improvement will further assure that the balloon fills uniformly, and that no portion of the balloon wall is highly stressed.

### Summary of the Invention

The present invention overcomes the above noted and other deficiencies in the prior art by providing an implantable band which is configured such that it will not fold or crease and, as a result, that will reliably and substantially fill with a filling solution.

In particular, the present invention provides an implantable band as recited in the claims.

The above summary of the present invention is not intended to describe each embodiment or every implementation of the present invention. Advantages and attainments, together with a more complete understanding of the invention, will become apparent and appreciated by referring to the following detailed description and claims taken in conjunction with the accompanying drawings.

### Brief Description of the Drawings

The accompanying drawings, which are incorporated in and constitute a part of this specification, illustrate embodiments of the invention, and, together with the general description of the invention given above, and the detailed description of the embodiments given below, serve to explain the principles of the present invention.
Figure 1 presents a pictorial view of a prior art gastric band and associated injection port;
Figure 2 is a cross-sectional view taken along line 2-2 in Figure 1;
Figure 3 is an isometric view of a removable gastric band embodying the present invention;
Figure 4 is a cross-sectional view taken along line 4-4 of Figure 3;
Figure 5 is a cross-sectional view taken along line 6-6 of Figure 4;
Figure 6 is a partial cross-sectional view similar to the cross-sectional view of Figure 5 showing an alternate location for the fluid supply tube;
Figure 7 is a cross-sectional view of an alternate embodiment of the fluid supply tube having a one-way check valve in the inlet of the fluid supply tube; and
Figure 8 is a side view of a gastric band which docs not fall within the scope of the claims, including a partial cut away of the balloon portion showing a cross-sectional view of a reinforced section thereof.

### Detailed Description of the Invention

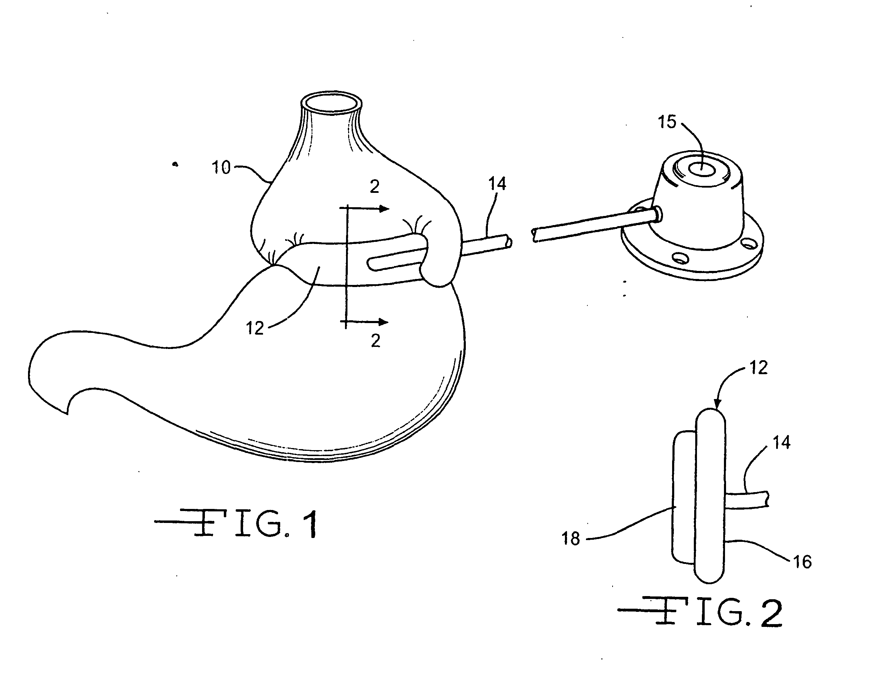

Referring now to the Figures wherein like numerals indicate like elements throughout, Figures 1 and 2 depict a stomach 10 having a fluidly inflatable, circumscribing band 12 as is known in the art. A fluid supply tube 14, fluidly communicating with band 12 is in fluid communication with a remotely located fluid injection port 15. Band 12 generally comprises an outer tension carrying belt having an inflatable balloon 18 affixed to the inside thereof. Balloon 18, when inflated, restricts the volume of stomach 10. To inflate balloon 18, a filling solution is injected into injection port 15 and the filling solution is conveyed to balloon 18 by way of supply tube 14.

The balloon 18 of the prior art band 12 has a tendency to fold and create creases when the band 12 is placed around the stomach 10. These creases may, in some instances, restrict flow of the filling solution to all areas of the balloon 18. This will affect the geometry of the balloon 18 and may also damage the balloon 18.

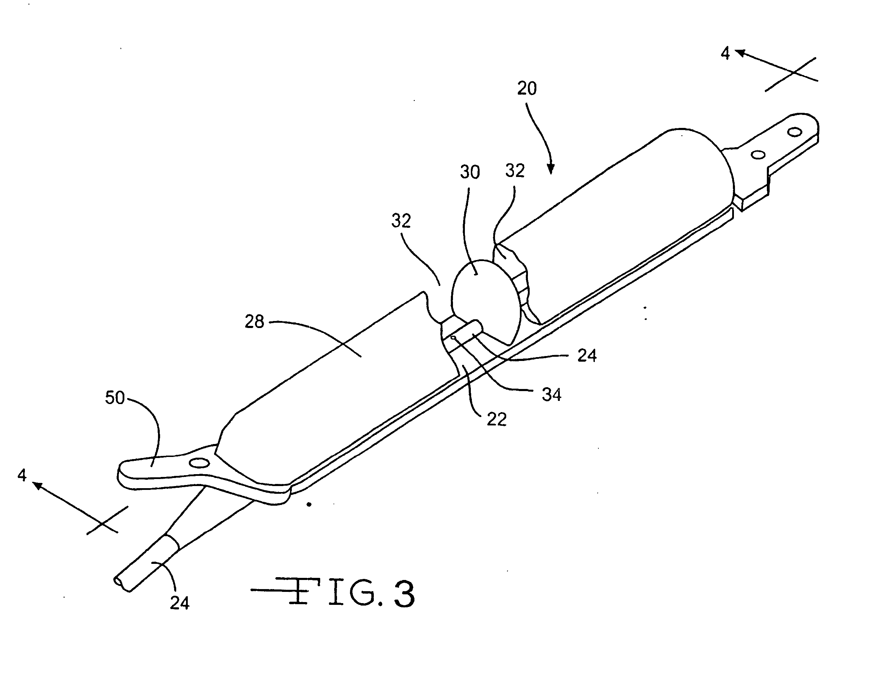

Referring now to Figures 3 through 5, the segmented gastric band 20 of the present invention is shown. Similar to prior art gastric bands, the segmented gastric band 20 has a tension carrying belt 22, a fluid supply tube 24 in fluid communication with a balloon 28 and a remotely located fluid injection port (not shown). The balloon 28 is preferably of a length of about 8cm to about 15 cm and more preferably about 11 cm; however, it should be appreciated that the balloon 28 may be of any length which would provide sufficient compression of the stomach. The balloon 28 is preferably comprised of material with a thickness between about 0.3cm and 0.7mm and more preferably about 0.5mm. Obviously, the thickness of the material is dependent on the balloon material and it should be appreciated that the thickness of the balloon may vary depending on the balloon composition. The tension carrying belt 22 is preferably longer than the balloon 28 and may be of any suitable length sufficient to accommodate the type of latching mechanism 50 employed. All components of segmented gastric band 20 are preferably comprised of medical grade silicone polymer but may be comprised of any flexible biocompatible material including implantable polyurethane.

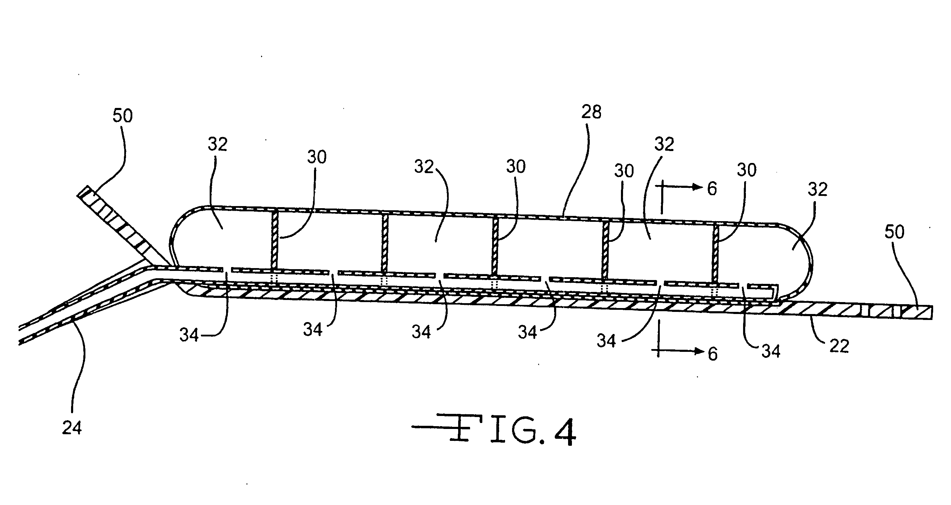

As best shown in Figure 4, the segmented gastric band 20 also has one or more partitions 30 located inside balloon 28, which separate the interior volume of balloon 28 into one or more chambers 32. The partitions may be evenly spaced within the balloon (as shown), unevenly spaced within the balloon, perpendicular to the longest edge of the tension carrying belt 22 or at an angle relative to the longest edge of the tension carrying belt 22. Also, partitions 30 may be reinforced with alternate material configurations or features such as multiple layers including one or more materials, thicker material, textured material, or a more dense material so as to help the balloon 28 bend at these locations.

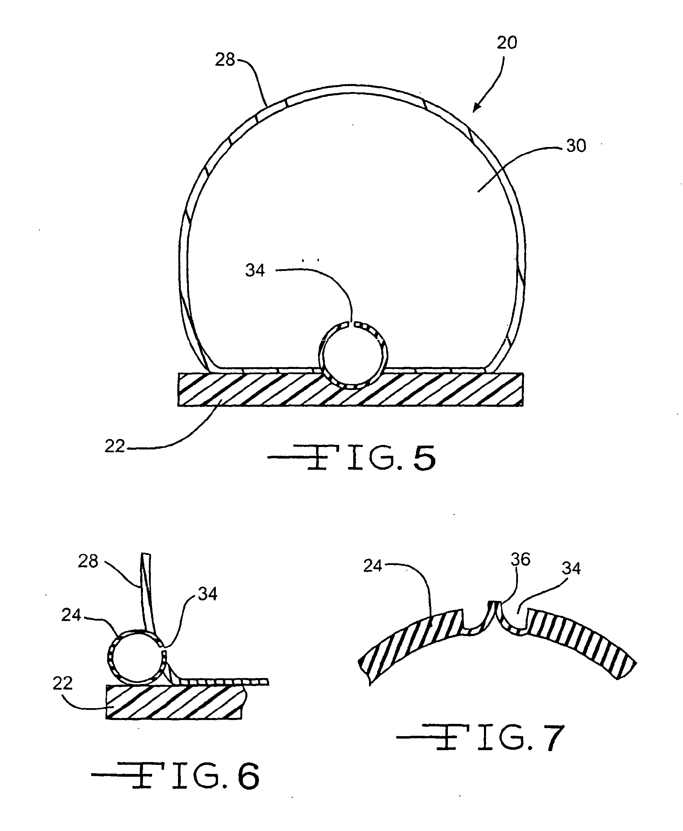

Fluid supply tube 24 has inlets 34 each of which correspond with and distribute the filling solution to each chamber 32. Inlets 34 may be of substantially identical diameters. Alternatively, inlets 34 may be ordered along the tube 24 from smallest diameter to largest diameter from end closest to fluid injection port such that the chamber closest to injection port fills with the filling solution at substantially the same rate as chambers further from the injection port. Also, in some applications, and as shown in Figure 7, it may be beneficial to place a one way check valve in inlet 34 such that the filling solution is allowed to enter the chamber but is not allowed to leave chamber via inlet 34. For example, Figure 7 shows fluid supply tube with inlet 34 including a duck bill type check valve 36 whereby the filling solution is allowed to enter chamber but is not allowed to escape via inlet 34. While a duck bill type check valve is shown in the illustrative embodiment, any type of one way check valve which would allow the filling solution to enter chamber and prevent the filling solution from escaping chamber via inlet would suffice. Alternatively, since, on occasion, the filling solution may need to be removed from the balloon, it may be advantageous to use a two-way check valve where the two-way check valve only allows the filling solution to leave the chambers when the pressure in the fluid supply tube becomes less than the pressure of the filling solution in the chambers. Preferably, this pressure differential would be created by using a syringe to withdraw fluid through the injection port.

The fluid supply tube 24 may be attached atop the surface of belt portion 22 inside balloon 28, or fluid supply tube 24 may be imbedded in belt portion 22 inside balloon 28, as shown in Figure 5. Fluid supply tube 24 may also be attached atop the surface of belt portion 22 outside of balloon 28, as shown in Figure 6, or fluid supply tube 24 may be imbedded in belt portion 22 outside of balloon 28.

Alternatively, each of the partitions may have a small opening which would allow the filling solution to flow from one chamber to the next via the opening. In this case, the fluid supply tube would only need an inlet to one chamber, preferably one of the chambers at either end of the balloon; although, multiple inlets could still be used.

The segmented gastric band 20 also includes a latching mechanism 50 so that the segmented gastric band may be releasably secured in an encircled position around a portion of the stomach. Latching mechanism 50 may be of any suitable configuration to hold segmented gastric band 50 in an encircled position such as, but not limited to, a guide tab and buckle configuration, a slide and channel configuration, a hook and eye configuration or, as shown in Figures 3, 4 and 8, tabs which are sutured together.

Installation of the segmented gastric band 20 is accomplished by first inserting the band into the patient's abdomen through a trocar. Next, a tunnel is created behind the stomach near the esophagogastric junction using a blunt dissection device. The segmented gastric band 20 is then grasped by an instrument, such as a grasper or blunt dissection device, and wrapped around the patient's stomach through the created tunnel. The latching mechanism 50 is then engaged. The injection port is then attached to the gastric band and the injection port is secured subcutaneously in the abdomen or other suitable location. A suitable filling solution, such as saline, is then injected into injection port whereby the solution is conveyed to chambers 32 of balloon 28 by way of inlets 34 in fluid supply tube 24. If necessary either at the time the gastric band is installed or at some time in the future, a predetermined quantity of the filling solution may be withdrawn for the balloon 28 by inserting a syringe into the injection port and withdrawing the solution.

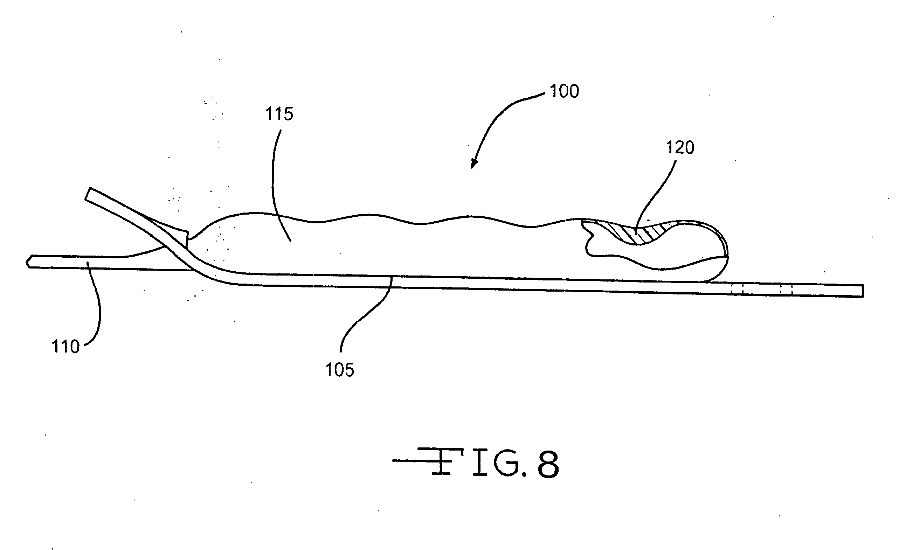

A gastric band which does not fall within the scope of the present invention is shown in Figure 8. In this embodiment the gastric band 100 similarly includes a tension carrying belt 105, a fluid supply tube 110 in fluid communication with a balloon 115 and a remotely located fluid injection port (not shown). In this embodiment, the balloon 115 has one or more reinforced sections 120. Preferably, the reinforced sections 120 are spaced evenly along the interior length of the balloon 115. Reinforced sections 120 may be comprised of a thicker material, textured material, a harder or softer material, or other similar configurations that would help balloon 115 bend between adjacent reinforced sections 120 when placed in an encircling position around stomach. In the preferred embodiment, shown in Figure 8, each end of each reinforced section 120 is substantially the same thickness as the balloon 115; however, each reinforced section is thickest,' and thicker than the balloon, at its midpoint. At the midpoint, each reinforced section 120 is preferably between about two and eight times the thickness of the balloon 115, and more preferably about four times the thickness of the balloon 115. Reinforced sections 120 may or may not be used to cause balloon 115 to segment into chambers when encircled around stomach.

While the present invention has been illustrated by the description of several embodiments and while the illustrative embodiments have been described in considerable detail, it is not the intention of the applicant to restrict or in any way limit the scope of the appended claims to such detail. Additional advantages and modifications may readily appear to those skilled in the art.

Further, it will become readily apparent to those skilled in the art that the above invention has equally applicability to other types of implantable bands. For example, bands are used for the treatment of fecal incontinence. One such band is described in U.S. Patent 6,461,292. Bands can also be used to treat urinary incontinence. One such band is described in U.S. Patent Application 2003/0105385. Bands can also be used to treat heartburn and/or acid reflux. One such band is described in U.S. Patent 6,470,892. Bands can also be used to treat impotence. One such band is described in U.S. Patent Application 2003/0114729.

## Claims

1. An implantable band (20) comprising:
a tension carrying belt (22);
a balloon (28) attached to said tension carrying belt (22),;
a fluid supply tube (24) in fluid communication with said balloon (28) and attached to said tension carrying belt (22);
a plurality of partitions (30) located inside said balloon (28), the plurality of partitions (30) defining a plurality of inner chambers (32); **characterised in that:**
said fluid supply tube (24) further comprises a plurality of inlets (34) wherein each of said plurality of inlets (34) is in fluid communication with a corresponding one of said plurality of inner chambers (32) of said balloon (28) and said fluid supply tube (24), wherein two or more of said plurality of inlets (34) of said fluid supply tube (24) are of different sizes, and said plurality of inlets (34) are ordered from smallest to largest along said fluid supply tube (24) such that said plurality of chambers (32) of said balloon (28) fill at substantially same rates.

2. The implantable band (20) of claim 1, wherein said fluid supply tube (24) further comprises a plurality of check valves (36) each of which is located within one of said plurality of inlets (34).

3. The implantable band (20) of claim 2, wherein each of said plurality of check valves (36) comprises a one-way check valve.

4. The implantable band (20) of claim 2 wherein each of said plurality of check valves (36) comprises a two-way check valve.

## Patentansprüche

1. Implantierbares Band (20), das aufweist:
einen Spannung haltenden Gurt (22);
einen Ballon (28), der an dem Spannung haltenden Gurt (22) befestigt ist;
eine Fluidzuführleitung (24) in fluidischer Verbindung mit dem Ballon (28) und an dem Spannung haltenden Gurt (22) befestigt;
eine Vielzahl von Trennwänden (30), die sich innerhalb des Ballons (24) befindet, wobei die Vielzahl der Trennwände (30) eine Vielzahl innerer Kammern (32) definiert; **dadurch gekennzeichnet, dass**
die Fluidzuführleitung (24) weiter eine Vielzahl von Einlässen (34) aufweist, wobei jeder aus der Vielzahl der Einlässe (34) in fluidischer Verbindung mit einer entsprechenden aus der Vielzahl der inneren Kammern (32) des Ballons (28) und der Fluidzuführleitung (24) steht, wobei zwei oder mehr aus der Vielzahl der Einlässe (24) der Fluidzuführleitung (24) eine unterschiedliche Größe haben und die Vielzahl der Einlässe (34) entlang der Fluidzuführleitung (24) vom kleinsten zum größten angeordnet ist, so dass die Vielzahl der Kammern (22) des Ballons (28) mit im Wesentlichen derselben Geschwindigkeit befüllt wird.

2. Implantierbares Band (20) nach Anspruch 1, bei dem die Fluidzuführleitung (24) weiter eine Vielzahl von Absperrventilen (36) aufweist, wobei sich jedes innerhalb einem der Vielzahl der Einlässe (34) befindet.

3. Implantierbares Band (20) nach Anspruch 2, bei dem jedes aus der Vielzahl der Absperrventile (36) ein Einweg-Absperrventil aufweist.

4. Implantierbares Band (20) nach Anspruch 2, bei dem jedes aus der Vielzahl der Absperrventile (36) ein Zweiwege-Absperrventil aufweist.

## Revendications

1. Bande implantable (20) qui comprend :
→ une ceinture de tension (22) ;
→ un ballon (28) fixé à ladite ceinture de tension (22) ;
→ un tube d'alimentation en fluide (24) en communication fluidique avec ledit ballon (28) et fixé à ladite ceinture de tension (22) ;
→ une pluralité de partitions (30) situées à l'intérieur dudit ballon (28), la pluralité de partitions (30) définissant une pluralité de chambres intérieures (32) ; **caractérisée en ce que :**
→ ledit tube d'alimentation en fluide (24) comprend en outre une pluralité d'orifices d'entrée (34), chacun des d'orifices d'entrée de ladite pluralité d'orifices d'entrée (34) étant en communication fluidique avec une chambre intérieure correspondante d'une pluralité de chambres intérieures (32) dudit ballon (28) et ledit tube d'alimentation en fluide (24), deux d'orifices d'entrée ou plus de ladite pluralité d'orifices d'entrée (34) dudit tube d'alimentation en fluide (24) étant de tailles différentes, et ladite pluralité d'orifices d'entrée (34) étant ordonnés du plus petit au plus grand le long dudit tube d'alimentation en fluide (24) de manière à ce que les chambres de ladite pluralité de chambres (32) dudit ballon (28) se remplissent sensiblement aux mêmes vitesses.

2. Bande implantable (20) selon la revendication 1, dans laquelle ledit tube d'alimentation en fluide (24) comprend en outre une pluralité de clapets anti-retour (36) étant chacun situé à l'intérieur de l'un des orifices d'entrée de ladite pluralité d'orifices d'entrée (34).

3. Bande implantable (20) selon la revendication 2, dans laquelle chacun des clapets anti-retour de ladite pluralité de clapets anti-retour (36) comprend un clapet anti-retour unidirectionnel.

4. Bande implantable (20) selon la revendication 2, dans laquelle chacun des clapets anti-retour de ladite pluralité de clapets anti-retour (36) comprend un clapet anti-retour bidirectionnel.
